# EUROPEAN PATENT SPECIFICATION

(11) **EP 4 395 719 B1**
(45) Date of publication and mention of the grant of the patent: **24.09.2025**
(21) Application number: 22777402.3
(22) Date of filing: 26.08.2022
(51) Int. Cl.: A61F 5/453

(54) **MALE EXTERNAL CATHETER INCLUDING NONWOVEN MATERIAL AND SPACER FABRIC**
EXTERNER MÄNNLICHER KATHETER MIT VLIESSTOFF UND ABSTANDSSTOFF
CATHÉTER EXTERNE MÂLE COMPRENANT UN MATÉRIAU NON TISSÉ ET TISSU D'ESPACEMENT

(30) Priority: 30.08.2021 US 202163238477 P
(43) Date of publication of application: 10.07.2024
(73) Proprietor: Purewick Corporation, El Cajon, California 92020 (US)
(72) Inventor: YIN, Zhihui, Lilburn, Georgia 30047 (US); MANN, Gregory, Covington, Georgia 30014 (US); ANDERSON, Michael, Monroe, Georgia 30655 (US)
(74) Representative: Studio Torta S.p.A.
(86) International application number: PCT/US2022/041688
(87) International publication number: WO 2023/034139

(56) References cited:
- WO-A1-2021/155206
- WO-A1-2022/098536
- WO-A2-2008/026106
- US-A1- 2004 236 292
- US-A1- 2008 091 153
- US-A1- 2012 296 305

## Description

### BACKGROUND

A person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, urinary catheters can be uncomfortable, painful, and can lead to complications, such as infections. Additionally, bed pans, which are receptacles used for the toileting of bedridden patients are sometimes used. However, bedpans can be prone to discomfort, spills, and other hygiene issues. Examples of fluid collection devices are disclosed in WO2021155206A1, US2012296305A1, the former disclosing a fluid collection device according to the preamble of claim 1.

### SUMMARY

The invention is set out in the appended set of claims.

Embodiments of fluid collection devices and systems are disclosed herein. In an embodiment, a fluid collection device includes a fluid impermeable barrier and a base secured to the fluid impermeable barrier. The fluid impermeable barrier at least partially defines a chamber within the fluid collection device and extends at least partially between a proximal end region of the fluid collection device and a distal end region of the fluid collection device. The base at least partially defines the chamber and an opening in fluid communication with the chamber. The base includes at least a first layer including at least a nonwoven fabric extending at least partially between the proximal end region and the distal end region.

In an embodiment, a method of manufacturing a fluid collection device is disclosed. The method includes securing a first layer to a second layer to form base, the first layer including at least a nonwoven fabric and the second layer including a fluid permeable multilayer. The method also includes securing a fluid impermeable barrier to the base to form a chamber in the fluid collection device at least partially defined by the fluid impermeable barrier and the second layer and in fluid communication with an opening at least partially defined by the base and sized to receive at least a portion of a penis therethrough.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a fluid collection system, according to an embodiment.
**FIG. 2A** is a top view of a fluid collection device, according to an embodiment.
**FIG. 2B** is a bottom view of the fluid collection device of **FIG. 2A****.**
**FIG. 3A** is a cross-sectional view of the fluid collection device of **FIG. 2A** taken along line 3A-3A in **FIG. 2A****.**
**FIG. 3B** is top view of a base of the fluid collection device of **FIG. 2A** with the fluid impermeable barrier removed, according to an embodiment.
**FIG. 3C** is an exploded view of the base of the fluid collection device of **FIG. 3B****,** according to an embodiment.
**FIG. 4** is a flow diagram of a method of manufacturing a fluid collection device, according to an embodiment.
**FIG. 5** is a cross-sectional view of a portion of a base of a fluid collection device, according to an embodiment.
**FIG. 6** is a cross-sectional view of a portion of a base of a fluid collection device, according to an embodiment.
**FIG. 7** is a cross-sectional view of a portion of a base of a fluid collection device, according to an embodiment.
**FIG. 8** is a cross-sectional view of a portion of a base of a fluid collection device, according to an embodiment.
**FIG. 9** is a cross-sectional view of a portion of a base of a fluid collection device, according to an embodiment.

### DETAILED DESCRIPTION

Embodiments of fluid collection devices, systems, and methods of using the same are disclosed herein. The fluid collection devices disclosed herein are configured to collect fluids from an individual. The fluids collected by the fluid collection devices may include urine, penile discharge, reproductive fluids, blood, sweat, or other bodily fluids. Fluid collection devices described herein may be used in fluid collection systems. An example fluid collection device includes a male-external catheter fluid collection device configured to cover users in the hospital or at home. In some embodiments, the fluid collection devices include a one-piece device having a fluid impermeable barrier secured to a base that includes an adhesive. Fluid collection devices described herein may be configured to use with a user having either a protruding penis or a buried penis in which the penis is covered by excess skin in the pubic area or scrotum. The base of the fluid collection device may include an opening sized to receive a penis therethrough to position at least a portion of the penis in a chamber between the fluid impermeable barrier and the base of the fluid collection device. The opening of the fluid collection device also may be positioned over a buried penis. Whether the penis of the user is protruding or buried, the adhesive on the base may form a seal against the user effective to prevent undesirable leaking of discharged fluids from the fluid collection device. Conventional male external catheters typically are not suitable for use with average penis size, small penis size, and buried penises. Conventional male external catheters typically also are not effective for use with patients lying on a bed in the hospital, and are prone to leaking in this positioned. The configuration of at least one, some, or all embodiments of fluid collection devices described herein provide the technical effect that results in effective use and fluid collection with users having small or buried penises, as well as user in different body positions (e.g., supine or sitting). In some embodiments, the base may include a layer of one or more nonwoven materials and a layer of three-dimensional (3D) fabric. The fluid collection device also is configured to secure to a conduit to connect the fluid collection device to a vacuum source for removal of discharged fluids from the fluid collection device.

The nonwoven materials in the base may provide venting properties to the fluid collection device such that a vacuum pressure of about 0 Pa (0 mmHg) to about 2666 Pa (20 mmHg) may be maintained on the fluid collection device for an extended period of time to withdraw fluids (e.g., urine) from the fluid collection device. The nonwoven material may include and inner surface of the base at least partially defining a chamber, and may provide skin contact comfortability to areas around penis of the user. In some embodiments, the chamber is defined by and positioned between the nonwoven material and the fluid impermeable barrier. In some embodiments, the nonwoven material may include a multilayer or multiportion material having a fibers or filaments bonding or securing fabric sheets or web structures.

The 3D fabric may be configured to keep the base dry during application and use of the fluid collection device, such that embodiments of the fluid collection device may remain in place on the user for up to about 3 to about 5 days. Accordingly, in some embodiments, the 3D fabric may include a fluid permeable multilayer or multiportion fabric including a spacer material between two spaced apart layers. The 3D fabric may include multilayer fluid permeable fabric at least partially. For example, the 3D fabric may include two woven fabric layers spaced apart and linked by spacer threads or yarns, such as a fibers. The fibers connecting the two woven fabric layers may include polyester or nylon fibers. The 3D fabric may include polyester spacer mesh fabric and/or scuba fabric. Examples of the 3D fabric may include one or more of a 3D spacer mesh, a 3D spacer fabric, a spacer fabric, a 3D air spacer mesh, an air spacer fabric, a neoprene scuba fabric, a sandwich scuba fabric, or any combination thereof.

The 3D fabric may be configured to capture fluid (e.g., urine) from the user and allow fluid to flow to a port and/or a conduit for removal from the fluid collection device. The 3D fabric also may be configured to support the urine collection device in a predetermined shape, generally planar, even under application of a vacuum to the interior region of the urine collection device. The 3D fabric also may prevent the fluid collection device from folding or kinking, and thus prevent fluid removal from being restricted due to the fluid collection device folding or kinking under application of the vacuum source. Accordingly, embodiments of the fluid collection device disclosed herein are suitable for use with a user in any one of a standing, sitting, or supine position. Exemplary fluid collection devices for use with the systems and methods herein are described in more detail below.

In some embodiments, the fluid collection device includes a male external catheter manufactured to include waterproof and/or microporous polyethylene/polypropylene nonwoven fabric, spacer fabric, polyethylene film, and a fitting tube secured together by welding low trauma gel-like adhesive. In at least one, some, or all embodiments of fluid collection devices described herein, the waterproof and/or microporous polyethylene/polypropylene nonwoven fabric and the spacer fabric include wicking materials that result in the technical effect of capturing and draining urine continuously from the fluid collection device while also keeping the fluid collection device in a dry and cool condition under a vacuum. The polyethylene film is clear in at least one, some, or all embodiments of fluid collection devices described herein, resulting in the technical effect of allowing a user or caregiver to check skin of the user while the fluid collection device is in use.

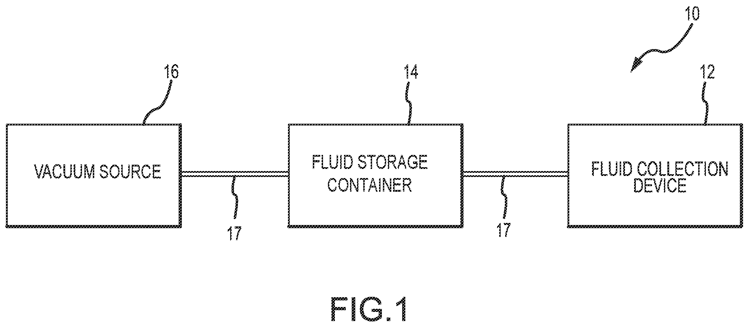

**FIG. 1** is a block diagram of a fluid collection system 10, according to an embodiment. The system 10 includes a fluid collection device 12 (*e.g.,* any of the fluid collection devices disclosed herein), a fluid storage container 14, and a vacuum source 16. The fluid collection device 10, the fluid storage container 14, and the vacuum source 16 may be fluidly coupled to each other via one or more conduits 17. For example, fluid collection device 10 may be operably coupled to one or more of the fluid storage container 14 or the vacuum source 16 via the conduit 17. Fluid (e.g., urine or other bodily fluids) collected in the fluid collection device 10 may be removed from the fluid collection device 10 via the conduit 17 secured to the fluid collection device 12. Suction force may be introduced into the chamber of the fluid collection device 12 via the inlet of the conduit 17 responsive to suction (e.g., vacuum) force applied at the outlet of the conduit 17.

The suction force may be applied to the outlet of the conduit 17 by the vacuum source 16 either directly or indirectly. The suction force may be applied indirectly via the fluid storage container 14. For example, the outlet of the conduit 17 may be disposed within the fluid storage container 14 and an additional conduit 17 may extend from the fluid storage container 14 to the vacuum source 16. Accordingly, the vacuum source 16 may apply suction to the fluid collection device 12 via the fluid storage container 14. The suction force may be applied directly via the vacuum source 16. For example, the outlet of the conduit 17 may be disposed within the vacuum source 16. An additional conduit 17 may extend from the vacuum source 16 to a point outside of the fluid collection device 12, such as to the fluid storage container 14. In such examples, the vacuum source 16 may be disposed between the fluid collection device 12 and the fluid storage container 14.

The fluid storage container 14 is sized and shaped to retain a fluid therein. The fluid storage container 14 may include a bag (*e.g.,* drainage bag), a bottle or cup (*e.g.,* collection jar), or any other enclosed container for storing bodily fluid(s) such as urine. In some examples, the conduit 17 may extend from the fluid collection device 12 and attach to the fluid storage container 14 at a first point therein. An additional conduit 17 may attach to the fluid storage container 14 at a second point thereon and may extend and attach to the vacuum source 16. Accordingly, a vacuum (*e*.*g*., suction) may be drawn through fluid collection device 12 via the fluid storage container 14. Fluid, such as urine, may be drained from the fluid collection device 12 using the vacuum source 16.

The vacuum source 16 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 16 may provide a vacuum or suction to remove fluid from the fluid collection device 12. In some examples, the vacuum source 16 may be powered by one or more of a power cord (*e.g.,* connected to a power socket), one or more batteries, or even manual power (*e.g.,* a hand operated vacuum pump). In some examples, the vacuum source 16 may be sized and shaped to fit outside of, on, or within the fluid collection device 12. For example, the vacuum source 16 may include one or more miniaturized pumps or one or more micro pumps. The vacuum sources disclosed herein may include one or more of a switch, a button, a plug, a remote, or any other device suitable to activate the vacuum source 16.

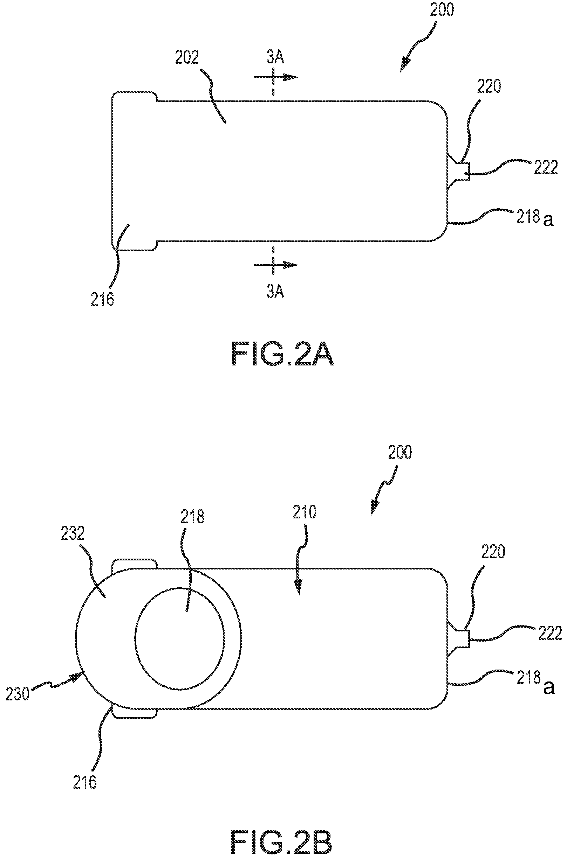

Turning now to **FIGS. 2A** and **2B****,** a fluid collection device 200 having a proximal end region 216 and a distal end region 218a may include fluid impermeable barrier 202 (shown in the top view of **FIG. 2A****)** and a base 210 (shown in the bottom view of **FIG. 2B****).** The fluid collection device 200 may be generally bag-like. In some embodiments, the fluid impermeable barrier 202 is substantially planar and substantially rectangular. In other embodiments, the fluid impermeable barrier 202 may include other geometric shapes and configurations. The fluid impermeable barrier 202 may include a longitudinal length greater than a lateral width of the fluid impermeable barrier 202. In some embodiments, the fluid impermeable barrier 202 may include a longitudinal length of about 10 cm to about 25 cm, about 10 cm to about 15 cm, about 12.5 cm to about 17.5 cm, about 15 cm to about 20 cm, about 17.5 cm to about 22.5 cm, about 20 cm to about 25 cm, at least about 10 cm, at least about 12.5 cm, at least about 15 cm, at least about 17.5 cm, at least about 20 cm, at least about 22.5 cm, or at least about 25 cm. In some embodiments, the fluid impermeable barrier 202 may include a lateral width of about 5 cm to about 15 cm, about 5 cm to about 8.5 cm, about 7 cm to about 10.5 cm, about 9 cm to about 12.5 cm, about 11 cm to about 14.5, less than about 14.5 cm, less than about 12.5 cm, less than about 10.5 cm, or less than about 8.5 cm.

The fluid impermeable barrier 202 may include a substantially flexible fluid impermeable material, such as a fluid impermeable polymer (*e*.*g*., silicone, polypropylene, polyethylene, polyethylene terephthalate, a polycarbonate, etc.), polyurethane films, thermoplastic elastomer, oil, another suitable material, or combinations thereof. In some embodiments, the fluid impermeable barrier includes a paper-like or bag-like fluid impermeable material or a fluid impermeable fabric. In at least one, some, or all embodiments, the fluid impermeable barrier 202 includes a polyethylene film or a polyurethane film that is substantially clear or transparent, effective to provide the technical effect of allowing a user or caregiver to check skin of the patient while the fluid collection device 200 is in use. In some embodiments, the fluid impermeable barrier 202 may include a thickness of about 0.03 to about 0.3 mm, about 0.03 mm to about 0.1 mm, about 0.04 mm to about 0.1 mm, about 0.05 mm to about 0.2 mm, or about 0.06 mm to about 3 mm.

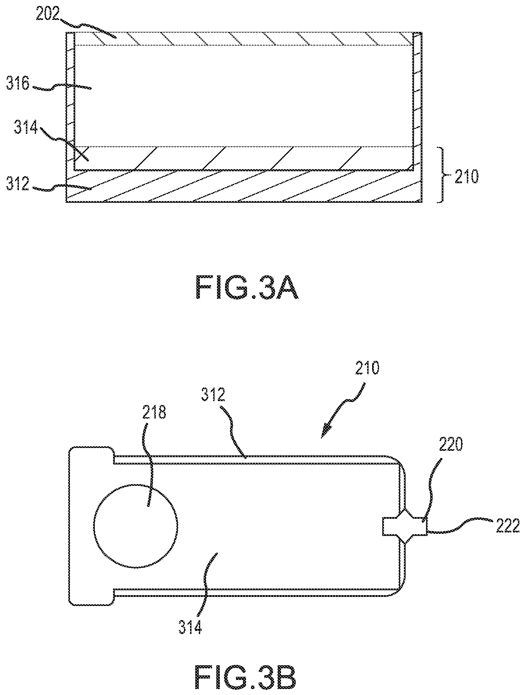

The fluid impermeable barrier 202 may be secured to the base 210 with, for example, an ultrasonic welding, thermal welding, radio frequency welding, microwave welding, a heat staking, a threading or seam, an adhesive, or combinations thereof. Turning to **FIG. 2B****,** the base may at least partially define an opening 218 or through hole extending therethrough. The opening 218 may be positioned proximate or near to the proximal end region 216 of the fluid collection device 216. In some embodiments, the opening 218 extends through both a first layer 312 and a second layer 314 (shown in **FIGS. 3A-3C** and described in greater detail below) such that the opening 218 is defined at least partially by both of the first layer 312 and the second layer 314 of the base 210. The opening 218 is sized and dimensioned to receive at least a portion of the penis therethrough. In some embodiments, the opening 218 is generally circular and includes a diameter of about 3.5 cm to about 9 cm, about 3.8 cm to about 5 cm, about 5 cm to about 6.4 cm, about 6.4 cm to about 7.6 cm, about 7.6 cm to about 8.9 cm, about 3.8 cm, about 5.1 cm, about 6.4 cm, or about 7.6 cm.

The fluid collection device 200 also may include securement element 230, according to an embodiment. The securement element 230 may be secured to the proximal end region 216 of the fluid collection device 200. For example, the securement element 230 may be secured to the proximal end region 216 of the base 210 with an ultrasonic welding, thermal welding, radio frequency welding, microwave welding, a heat staking, a threading or seam, an adhesive, or combinations thereof. In some embodiments, the securement element 230 is connected directly to the base 210. The securement element 230 may extend at least partially (e.g., entirely) around the opening 218. For example, the securement element 230 may be a generally annular securement element 230. The securement element 230 and the base 210 are connected such that the when the securement element 230 is secured to the user, the securement element 230 forms an at least partial seal between the chamber 316 (shown in **FIG. 3A****)** and the exterior, ambient environment of the fluid collection device 200. With the securement element 230 forming a seal, fluid discharged into the chamber 316 by a urethra either positioned in the chamber or buried on the user beneath the chamber 316 remains in the chamber 316 for removal through the aperture 222.

The securement element 230 may include an adhesive surface 232 configured to adhere the securement element 230 to the skin of the user around the penis. The adhesive surface 232 of the securement element 230 may be positioned such that the adhesive surface 232 adheres only to the skin of the user around the penis, effective to enable the fluid collection device to cover either a protruding penis or a buried penis. The adhesive surface 232 may be positioned on the securement element 230 generally opposite to the base 210 and/or port 220 of the fluid collection device 200. The adhesive surface 232 may be positioned at least partially (e.g., entirely) around the opening 218. The adhesive surface 232 may include one or more of a silicone gel adhesive, an acrylate/acrylic adhesive, an acrylate/acrylic gel adhesives, a hydrogel adhesive, or any combination thereof. On stainless steel or on low density polyethylene under degree peel testing, the adhesion strength of the adhesive surface 232 may be about 43 N/m (0.25 lb/in) to about 525 N/m (3 lb/in), about 43 N/m (0.4 lb/in) to about 437 N/m (2.5 lb/in), about 70 N/m (0.4 lb/in) to about 175 N/m (1 lb/in), about 175 N/m (1 lb/in) to about 263 N/m (1.5 lb/in), about 263 N/m (1.5 lb/in) to about 350 N/m (2 lb/in), about 350 N/m (2 lb/in) to about 437 N/m (2.5 lb/in), at least about 70 N/m (0.4 lb/in), at least about 175 N/m (1 lb/in), at least about 263 N/m (1.5 lb/in), at least about 350 N/m (2 lb/in), at least about 437 N/m (2.5 lb/in), less than about 70 N/m (0.4 lb/in), less than about 175 N/m (1 lb/in), less than about 263 N/m (1.5 lb/in), less than about 350 N/m (2 lb/in), or less than about 437 N/m (2.5 lb/in).

The fluid collection device 200 also may include a port 220 secured or connected to the base 210 at the distal end region 218a. The port 220 may define or otherwise include an aperture 222 configured to receive or secure to a tube or a conduit effective to provide fluid communication between the chamber 316 and a vacuum source. In some embodiments, the port 220 includes a protruding port sized and dimensioned to be inserted into the tube or conduit to friction fit the port 220 to the tube or conduit. In some embodiments, the port 220 may include a polyethylene fitting tube. In some embodiments, the port 220 may be formed of any suitable fluid impermeable material(s), such as a fluid impermeable polymer (e.g., silicone, polypropylene, polyethylene, polyethylene terephthalate, a polycarbonate, a thermoplastic elastomer, polyvinyl chloride, ethylene vinyl acetate, polytetrafluoroethylene etc.), a metal film, natural rubber, another suitable material, or combinations thereof. The port 220 may positioned between the fluid impermeable barrier 202 and the base 210 and in fluid communication with the chamber 316. In some embodiments, the port 220 is welded to at least one of the fluid impermeable barrier 202 and the base 210 between the fluid impermeable barrier 202 and the base 210.

When being worn by the user 50, the fluid collection device 200 may include a tube or conduit (not shown) detachably or fixedly secured to the port 220 of the fluid collection device 200. The conduit may include a flexible material such as plastic tubing (e.g., medical tubing). Such plastic tubing may include a thermoplastic elastomer, polyvinyl chloride, ethylene vinyl acetate, polytetrafluoroethylene, etc., tubing. In some embodiments, the conduit may include silicone or latex. The conduit provides fluid communication between an interior region (*e.g.,* the chamber 316) of the fluid collection device 200, a fluid storage container (not shown), and/or a portable vacuum source (not shown). For example, the conduit may directly or indirectly fluidly couple the interior region of the fluid collection device with the fluid storage container or the portable vacuum source.

Turning now to **FIG. 3A****,** a cross-sectional view of the fluid collection device 200 of **FIG. 2A** taken along line 3A-3A is illustrated, according to an embodiment. The base 210 of the fluid collection device 200 may at least partially define the chamber 316 (interior region) sized and dimensioned to receive at least a portion (*e*.*g*., most) of a penis of the user therein. In some embodiments, the chamber 316 is defined by and positioned directly between the fluid impermeable barrier 202 and the base 210 (*e.g.,* the second layer 314). The chamber 316 and/or the base 210 may include approximately any of the dimensions of the fluid impermeable barrier 202 described above. In some embodiments, the base 210 is generally rectangular and/or substantially planar. The base 210 in at least one, some, or all embodiments includes wicking materials that result in the technical effect of capturing and draining urine to the port 220 continuously, while also keeping the fluid collection device dry and cool under a vacuum applied through the port 220.

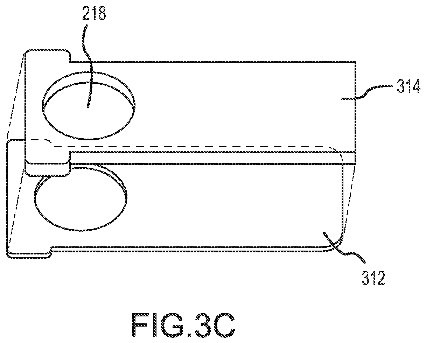

**FIG. 3B** illustrates the base 210 separated from the fluid impermeable barrier 202 and **FIG. 3C** shows an exploded view of the base 210. In some embodiments, the base 210 includes at least the first layer 312 including at least a nonwoven fabric and the second layer 314 including a 3D fabric or material. The second layer 314 is at least partially positioned between the first layer 312 and the chamber 316. For example substantially all of the first layer 312 may be spaced from the chamber 316 by the second layer 314. In some embodiments, the first layer 312 and the second layer 314 may extend at least partially between the proximal end region 216 and the distal end region 218a of the fluid collection device 200. The first layer 312 and the second layer 314 may be secured together with, for example, an ultrasonic welding, thermal welding, radio frequency welding, microwave welding, a heat staking, a threading or seam, an adhesive, or combinations thereof. In some embodiments, the second layer 314 is secured directly to the first layer 312 and the fluid impermeable barrier 202 is secured to directly to the first layer 312 with the second layer 314 positioned between first layer 312 and the fluid impermeable barrier 202. Returning to the cross-sectional view of **FIG. 3A****,** the first layer 312 may be generally U-shaped (with a planar bottom) and the second layer 314 may fit within a channel formed between two sidewalls of the first layer 312. In these and other embodiments, the fluid impermeable barrier 202 may be secured to the two sidewalls of the first layer 312 generally opposite to the bottom of the first layer 312 such that the chamber 316 is defined by the fluid impermeable barrier 202, the second layer 314, and portions of each of the two sidewalls of the first layer 312. In other embodiments, the sidewalls are absent from the first layer 312 and the fluid impermeable barrier 202 is secured to an outer perimeter or periphery of the first layer 312.

The nonwoven fabric of the first layer 312 may include sheet or web structures bonded together by entangling fiber or filaments and/or by perforating films. The sheet or web structures of the nonwoven fabric layer of the first layer 312 may be bonded together by one or more of mechanically, thermally, or chemically. In some embodiments, the first layer may include waterproof microporous polyethylene/polypropylene fabric. The nonwoven fabric of the first layer 312 may include flat or tufted porous sheets that are made directly from separate fibers, molten plastic, and/or plastic film. In some embodiments, the nonwoven fabric of the first layer 312 may include on or more of spunbonded polypropylene nonwoven fabric, spunbonded polyester fabric, spunbonded melt blown spunbonded polypropylene nonwoven fabric from BERRY^{™}. The first layer 312 including at least the nonwoven fabric may provide skin contact comfortability while the fluid collection device 200 is applied. For example, in some embodiments, if the first layer 312 is pressed against the area around the penis or other parts of the body, the nonwoven fabric may provide a comfortable, soft interface against those areas or parts of the body. The first layer 312 including at least the nonwoven fabric also may be configured to provide venting and/or waterproof properties to the base 210 that allow the fluid collection device 200 to maintain the vacuum condition in a closed system under vacuum operation to withdraw fluids from the fluid collection device 200. Any embodiments of the first layer 312 including at least the nonwoven fabric described herein may be configured to provide venting to the chamber 316 when a vacuum force is exerted or pulled on the chamber 316 through the aperture 222. For example, the first layer 312 may be configured to provided venting to the chamber 316 when a vacuum force of about 0 Pa (0 mmHg) to about 4000 Pa (30 mmHg) is pulled on chamber 316 through the aperture 222, such as a vacuum force of about 0 Pa (0 mmHg) to about 2000 Pa (15 mmHg), about 2000 Pa (15 mmHg) to about 4000 Pa (30 mmHg), about 0 Pa (0 mmHG) to about 1333 Pa (10 mmHg), about 1333 Pa (10 mmHg) to about 2666 Pa (20 mmHg), about 2666 Pa (20 mmHg) to about 4000 Pa (30 mmHG), at least about 667 Pa (5 mmHg), at least about 1333 Pa (10 mmHg), at least about 2000 Pa (15 mmHg), at least about 2666 Pa (20 mmHg), at least about 3333 Pa (25 mmHg), less than about 667 Pa (5 mmHg), less than about 1333 Pa (10 mmHg), less than about 2000 Pa (15 mmHg), less than about 2666 Pa (20 mmHg), less than about 3333 Pa (25 mmHg), or less than about 4000 Pa (30 mmHg).

One or more embodiments of the first layer 312 including at least the nonwoven fabric described herein may include pores. The pores may be spread generally uniformly through the first layer 312, or may be substantially isolated in one or more of the portions of the first layer 312a, 312b, 312c, or 312d described in greater detail below. The pores of the first layer 312 may by sized about 0.1 µm to about 5 µm, about 0.1 µm to about 1 µm, about 1 µm to about 2 µm, about 2 µm to about 3 µm, about 3 µm to about 4 µm, about 4 µm to about 5 µm, at least about 0.1 µm, at least about 1 µm, at least about 2 µm, at least about 3 µm, at least about 4 µm, less than about 1 µm, less than about 2 µm, less than about 3 µm, less than about 4 µm, or less than about 5 µm.

In some embodiments, the first layer 312 includes one or more of spunbonded polypropylene and/or melt blown polypropylene. In embodiments, in which the first layer 312 having one or more of spunbonded polypropylene or melt blown polypropylene, the first layer 312 may include a density of about 10 grams per square meter (gsm or g/m²) to about 30 gsm, about 12 gsm to about 25 gsm, about 12 gsm to about 18.5 gsm, about 18.5 gsm to about 25 gsm, about 10 gsm to about 20 gsm, about 20 gsm to about 30 gsm, about 10 gsm to about 15 gsm, about 15 gsm to about 20 gsm, about 20 gsm to about 25 gsm, about 25 gsm to about 30 gsm, at least about 10 gsm, at least about 15 gsm, at least about 20 gsm, at least about 25 gsm, less than about 15 gsm, less than about 20 gsm, less than about 25 gsm, or less than about 30 gsm.

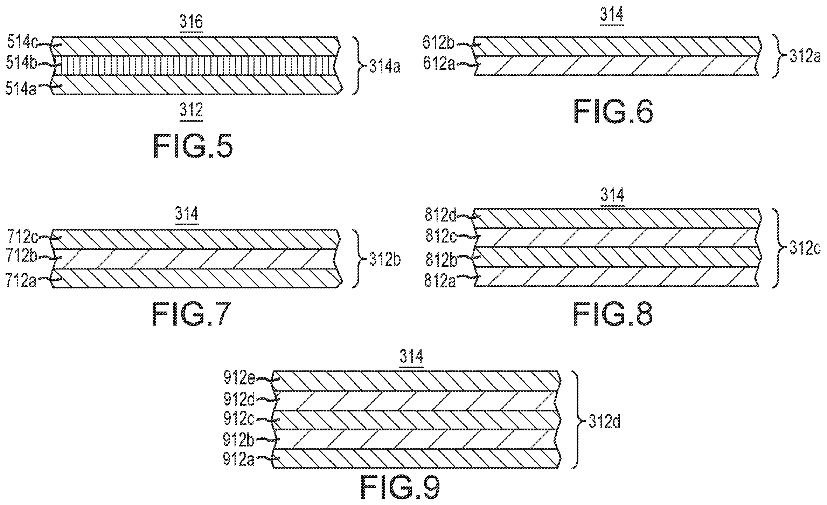

In some embodiments, the first layer 312 including at least the nonwoven fabric may include multiple portions or layers. Each portion of the first layer may include nonwoven material. For example, turning to **FIG. 6****,** the first layer 312a may include a first outer portion 612a and a first inner portion 612b. In some embodiments, the first inner portion 612b may be adjacent to and/or interface the second layer 314. The first outer portion 612a may define at least a portion of an outer surface of the base 210. In some embodiments, an additional layer may cover the first outer portion 612a and define at least a portion of the outer surface of the base 210. The materials of the first outer portion 612a and the first inner portion 612b of the first layer 312a may vary according to different embodiments. In some examples, the first layer 312a includes a spunbonded fabric including polyethylene and polypropylene. Embodiments of the first layer 312a including the spunbonded fabric including the polyethylene and polypropylene may include the first outer portion 612a and the first inner portion 612b. For example, in some embodiments, the first outer portion 612a includes a polyethylene microporous film and the first inner portion 612b includes a nonwoven polypropylene. The polyethylene microporous film of the first outer portion 612a may include a nonwoven polyethylene microporous film.

Turning to **FIG. 7****,** in some embodiments, the first layer 312b may include a first outer portion 712a, a first inner portion 712c, and a first intermediate portion 712b between the first inner portion 712c and the first outer portion 712a. In some embodiments, each of the first inner portion 712c, the first outer portion 712a, and the first intermediate portion 712a may include nonwoven material. In some embodiments, each of the first inner portion 712c and the first outer portion 712a may include nonwoven material. The first inner portion 712c may be adjacent to or interfacing the second layer 314. The first outer portion 712a may define at least a portion of an outer surface of the base 210. In some embodiments, an additional layer may cover the first out portion 712a and define at least a portion of the outer surface of the base 210. Similar to the first layer 312a, the first layer 312b may include a spunbonded fabric including polyethylene and polypropylene. For example, the first layer 312b may include the first inner portion 712c having a nonwoven polypropylene material, the first outer portion 712a having a polypropylene material (such as a nonwoven polypropylene material), and the first intermediate portion 712b having polyethylene (such as a nonwoven polyethylene) between the first inner portion 712c and the first outer portion 712a.

Embodiments of the first layer 312a or 312b including the spunbonded fabric including the polyethylene and polypropylene also may have a density of about 20 gsm to about 100 gsm, such as about 20 gsm to about 60 gsm, about 60 gsm to about 80 gsm, about 80 gsm to about 100 gsm, about 20 gsm to about 30 gsm, about 30 gsm to about 40 gsm, about 40 gsm to about 50 gsm, about 50 gsm to about 60 gsm, about 60 gsm to about 70 gsm, about 70 gsm to about 80 gsm, about 80 gsm to about 90 gsm, about 90 gsm to about 100 gsm, at least about 20 gsm, at least about 30 gsm, at least about 40 gsm, at least about 50 gsm, at least about 60 gsm, at least 70 gsm, at least about 80 gsm, at least about 90 gsm, at least about 100 gsm, less than about 20 gsm, less than about 30 gsm, less than about 40 gsm, less than about 50 gsm, less than about 60 gsm, less that about 70 gsm, less than about 80 gsm, less than about 90 gsm, or less than about 100 gsm.

Returning to **FIG. 6****,** in some embodiments, materials of the first layer 312a may include a polyester material and a polyethylene material. For example, the first layer 312a may include a first outer portion 612a including a polyethylene material (such as a nonwoven polyethylene material) and a first inner portion 612b including a polyester material (such as a nonwoven polyester material). In embodiments of the first layer 312a including a polyester material and a polyethylene material, the first layer 312a may include a density of about 20 gsm to about 120 gsm, such as about 20 gsm to about 60 gsm, about 60 gsm to about 80 gsm, about 80 gsm to about 100 gsm, about 100 gsm to about 120 gsm, about 20 gsm to about 30 gsm, about 30 gsm to about 40 gsm, about 40 gsm to about 50 gsm, about 50 gsm to about 60 gsm, about 60 gsm to about 70 gsm, about 70 gsm to about 80 gsm, about 80 gsm to about 90 gsm, about 90 gsm to about 100 gsm, about 100 gsm to about 110 gsm, about 110 gsm to about 120 gsm, at least about 20 gsm, at least about 30 gsm, at least about 40 gsm, at least about 50 gsm, at least about 60 gsm, at least 70 gsm, at least about 80 gsm, at least about 90 gsm, at least about 100 gsm, at least about 110 gsm, at least about 120 gsm, less than about 20 gsm, less than about 30 gsm, less than about 40 gsm, less than about 50 gsm, less than about 60 gsm, less that about 70 gsm, less than about 80 gsm, less than about 90 gsm, less than about 100 gsm, less than about 110 gsm, or less than about 120 gsm.

In some embodiments, materials of the first layer 312a may include spunbonded fabric including a cellulose material and a polyethylene material. For example, the first layer 312a may be spunbonded and include a first outer portion 612a including a polyethylene material and a first inner portion 612b including a cellulose material. In some embodiments, only one of the first outer portion 612a including the polyethylene material and the first inner portion 612b including a cellulose material is spunbonded. In some embodiments, both of the first outer portion 612a including the polyethylene material and the first inner portion 612b including a cellulose material is spunbonded.

Turning again to **FIG. 7****,** in some embodiments, the first layer 312b may include spunbonded polypropylene and melt blown polypropylene. Nonwoven polypropylene fabric may be manufactured through a melt blow process and may, in some example, include only polypropylene. The first outer portion 712a and the first inner portion 712c may both include a spunbonded polypropylene, and the first intermediate portion 712b may include melt blown polypropylene between the first inner portion 712c and the first outer portion 712b. In embodiments of the first layer 312b including spunbonded polypropylene and melt blown polypropylene, the density of the first layer may be about 10 gsm to about 60 gsm, about 10 gsm to about 35 gsm, about 35 gsm to about 60 gsm, about 10 gsm to about 20 gsm, about 20 gsm to about 30 gsm, about 30 gsm to about 40 gsm, about 40 gsm to about 50 gsm, about 50 gsm to about 60 gsm, at least about 10 gsm, at least about 20 gsm, at least about 30 gsm, at least about 40 gsm, at least about 50 gsm, at least about 60 gsm, less than about 20 gsm, less than about 30 gsm, less than about 40 gsm, less than about 50 gsm, or less than about 60 gsm.

In some embodiments, the first layer 312 may include one or more of nonwoven spunbonded polypropylene, nonwoven polyethylene, and/or nonwoven melt blown polypropylene. Turning ahead in the drawings to **FIG. 8****,** according to some embodiments, the first layer 312c may include a first outer portion 812a, a first inner portion 812d, a first intermediate portion 812c, and a second intermediate portion 812b. The first inner portion 812d may be adjacent to or interfacing the second layer 814. The first outer portion 812a may define at least a portion of an outer surface of the base 210. In some embodiments, an additional layer may cover the first out portion 812a and define at least a portion of the outer surface of the base 210. The first inner portion 812d may include nonwoven spunbonded polypropylene and the first outer portion 812a may include nonwoven polyethylene. The first intermediate portion 812c may be adjacent to the first inner portion 812d and may include nonwoven melt blown polypropylene. The second intermediate portion 812b may be between the first intermediate portion 812c and the first outer portion 812a and may include nonwoven spunbonded polypropylene. The first layer 312c may include a density of about 20 gsm to about 90 gsm, such as about 25 gsm to about 85 gsm, about 20 gsm to about 55 gsm, about 55 gsm to about 90 gsm, about 25 gsm to about 35 gsm, about 35 gsm to about 45 gsm, about 45 gsm to about 55 gsm, about 55 gsm to about 65 gsm, about 65 gsm to about 75 gsm, about 75 gsm to about 85 gsm, at least about 20 gsm, at least about 30 gsm, at least about 40 gsm, at least about 50 gsm, at least about 60 gsm, at least about 70 gsm, at least about 80 gsm, less than about 25 gsm, less than about 35 gsm, less than about 45 gsm, less than about 55 gsm, less than about 65 gsm, less than about 75 gsm, less than about 85, or less than about 95 gsm.

In some embodiments, the first layer 312 may include one or more of nonwoven spunbonded polypropylene, nonwoven polyethylene, and/or nonwoven melt blow polypropylene. Turning ahead in the drawings to **FIG. 9****,** according to some embodiments, the first layer 312d may include a first outer portion 912a, a first inner portion 912e, a first intermediate portion 912d, a second intermediate portion 912c, and a third intermediate portion 912b. The first inner portion 912e may be adjacent to or interfacing the second layer 314. The first outer portion 912a may define at least a portion of an outer surface of the base 210. In some embodiments, an additional layer may cover the first out portion 912a and define at least a portion of the outer surface of the base 210. The first outer portion 912a may include nonwoven polyethylene and the first inner portion may include nonwoven spunbonded polypropylene. The first intermediate portion 912d may be adjacent to or interfacing the first inner portion 912e and may include nonwoven melt blown polypropylene. The second intermediate portion 912c may be adjacent to the first intermediate portion 912d and may include nonwoven spunbonded polypropylene. The third intermediate portion 912b may be positioned between the second intermediate portion 912c and the first outer portion 912a and may include melt blown polypropylene.

Returning in the drawings to **FIG. 3A****,** the base 210 of the fluid collection device 200 includes the second layer 314 that may define at least a portion of the chamber 316. The second layer 314 may include a 3D or multilayer fluid permeable fabric at least partially positioned between the first layer 312 and the chamber 316. In some embodiments, the chamber 316 is positioned between and at least partially defined by the fluid impermeable barrier 202 and the second layer 314. The 3D fabric of the second layer 314 may include two woven fabric layers spaced apart and linked by spacer threads or yarns, such as a fibers. The fibers connecting the two woven fabric layers may include polyester or nylon fibers. The 3D fabric of the second layer 314 may include polyester spacer mesh fabric and/or scuba fabric. In some embodiments, the second layer 314 may include a second inner portion including one or more of non-wove polyester, polypropylene, and/or polyethylene, and a second outer portion including one or more of polyester or nylon. The 3D fabric may be fluid permeable and configured to capture fluid (*e*.*g*., urine) from the user and/or direct fluid to the aperture 222 for removal from the fluid collection device 200. The second layer 314 including the 3D fabric, then, may provide the fluid collection device 200 with urine capture capability and may assist in the drainage or removal of fluids from the fluid collection device 200 under a vacuum force. For example, when a user is wearing the fluid collection device 200, urine may enter the chamber 316 and then flow through the second layer 314 to the aperture 222 for removal from the fluid collection device 200. In some embodiments, the second layer 314 may wick and/or otherwise allow transport of the urine to the aperture 222. The permeable properties referred to herein may be wicking, capillary action, diffusion, or other similar properties or processes, and are referred to herein as "permeable" and/or "wicking." Such "wicking" may exclude absorption into the wicking material. While no absorption is desired, the term "substantially no absorption" may allow for nominal amounts of absorption of fluid into the wicking material (*e*.*g*., absorbency) of the second layer 314, such as less than about 30 wt% of the dry weight of the fluid permeable body 110, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the second layer 314. In an embodiment, the second layer 314 may include at least one absorbent or adsorbent material. The second layer 314 may include a one-way fluid movement fabric.

The 3D fabric also may be configured to support the base 210 of the fluid collection device 200 in a predetermined shape, generally planar. For example, in some embodiments, when a vacuum is applied to the chamber 316 of the base 210 via the port 220, the 3D fabric of the second layer 314 is configured to support the base 210 in a generally planar shape. The second layer 314 including the 3D fabric, then, may prevent or inhibit the fluid restriction that occurs if a fluid collection device kinks when a vacuum is being pulled on a chamber. The second layer 314 also may provide relative drying conditions for a user, thus allowing more long term application of the fluid collection device 200, such as about 3 to about 5 days. Examples of the second layer 314 may include a 3D spacer mesh, a 3D spacer fabric, a spacer fabric, a 3D air spacer mesh, an air spacer fabric, a neoprene scuba fabric, a sandwich scuba fabric, or any combination thereof.

The second layer 314 may include a thickness of about 0.5 mm to about 10 mm, about 1 mm to about 6 mm, about 1.5 mm to about 4.5 mm, about 4.5 mm to about 8.5 mm, about 6 mm to about 10 mm, about 1 mm to about 3mm, about 3 mm to about 5 mm, about 5 mm to about 7 mm, about 7 to about 9 mm, at least about 0.5 mm, at least about 1 mm, at least about 2 mm, at least about 3 mm, at least about 4 mm, at least about 5 mm, at least about 6 mm, at least about 7 mm, at least about 8 mm, at least about 9 mm, less than about 1 mm, less than about 2 mm, less than about 3 mm, less than about 4 mm, less than about 5 mm, less than about 6 mm, or less than about 7 mm.

In some embodiments, the second layer 314 may include multiple layers or portions. For example, turning ahead in the drawings to **FIG. 5****,** a second layer 314a may include a second outer portion 514a and at least a second inner portion 514c. The second inner portion 514c may at least partially define the chamber 316 such that the chamber 316 is positioned at least partially (*e.g.,* directly) between the fluid impermeable barrier 202 and the second inner portion 514C. In more particular examples, the second layer 314a may include the second outer portion 514a, the second inner portion 514c, and an intermediate portion 514b positioned between and/or connecting the second inner portion 514c and the second outer portion 514a. In some embodiments, the intermediate portion 514b may include fibers or a fiber material. For example, the fibers of the intermediate portion 514b may include spacer yarns connecting the second inner out portion 514a and the second inner portion 514c.

In some embodiments, the second layer 314 includes a spacer fabric configured to capture fluid and help drain the fluid from the fluid collection device 200 under a vacuum. The spacer fabric also may act as a supporting material to help hold the shape of the fluid collection device 200. The spacer fabric of the second layer 314 may include the second inner portion 514c, the second outer portion 514a, and fibers of the intermediate portion 514b connecting the second inner portion 514c and the second outer portion 514a. In some embodiments, the spacer fabric of the second layer 314 may include a polyester spacer fabric. For example, at least the second inner portion 514c and the second outer portion 514a of the second layer 314 may include and/or consist essentially of a polyester material. In some examples of the second layer 314 including a spacer fabric having polyester or other materials, the second inner portion 514c may include an inner mesh portion having holes or openings of a first size and the second outer portion 514a may include an outer mesh portion having holes of a second size smaller than the holes of the inner mesh portion having the holes of the first size. In some examples of the second layer 314 including a spacer fabric having polyester or other materials, the second inner portion 514c may include a polyester fabric and the second outer portion 514a may include a polyester mesh material having a plurality of holes or openings. In some examples of the second layer 314 including a spacer fabric having polyester or other materials, the second inner portion 514c may include a cotton fabric and the second outer portion may include a polyester mesh having a plurality of holes or openings.

The second layer 314 including the spacer fabric may include a density of about 150 gsm to about 300 gsm, about 190 gsm to about 260 gsm, about 150 gsm to about 190 gsm, about 190 gsm to about 230 gsm, about 230 gsm to about 260 gsm, about 260 gsm to about 300 gsm, about 190 gsm to about 200 gsm, about 200 gsm to about 210 gsm, about 210 gsm to about 220 gsm, about 230 gsm to about 240 gsm, about 240 gsm to about 250 gsm, about 250 gsm to about 260 gsm, at least about 190 gsm, at least about 200 gsm, at least about 210 gsm, at least about 220 gsm, at least about 230 gsm, at least about 240 gsm, at least about 250 gsm, at least about 260 gsm, less than about 190 gsm, less than about 200 gsm, less than about 210 gsm, less than about 220 gsm, less than about 230 gsm, less than about 240 gsm, less than about 250 gsm, or less than about 260 gsm.

In some embodiments, the second layer 314 includes a scuba fabric. The scuba fabric of the second layer 314 may include a neoprene or polychloroprene material. In some embodiments, the scuba fabric includes a polyester-spandex knit fabric. For example, the scuba fabric of the second layer 314 may include about 10 wt% to about 25 wt% neoprene or polychloroprene material. The scuba fabric of the second layer 314 may include one or more of the second inner portion 514c, the second outer portion 514a, or the intermediate portion 514b connecting the second inner portion 514c and the second outer portion 514a. In some embodiments, the second outer portion 514a includes a polyester fabric, the second inner portion 514c includes a polyester fabric, and the intermediate portion 514b includes the scuba fabric including the neoprene. In some embodiments, at least one (*e.g.,* both) of the second outer portion 514a and the second inner portion 514c includes the scuba fabric. The intermediate portion 514b may include fibers such as spacer yarns connecting the second outer portion 514b and the second inner portion 514c when the second outer portion 514a and the second inner portion 514c include the scuba fabric.

In some embodiments, the second outer portion 514a includes a polyester fabric, the second inner portion 514c includes a cotton fabric, and the intermediate portion 514b includes the scuba fabric including the neoprene. In some embodiments, at least one (*e.g.,* both) of the second outer portion 514a and the intermediate portion 514b may include scuba fabric and the second inner portion 514c may include cotton fabric. The intermediate portion 514b may include fibers such as spacer yarns connecting the second outer portion 514b and the second inner portion 514c when the second outer portion 514a includes scuba fabric and the second inner portion 514c includes cotton fabric.

The second layer 314 including the scuba fabric may include a density of about 225 gsm to about 400 gsm, about 250 gsm to about 380 gsm, about 250 gsm to about 300 gsm, about 300 gsm to about 350 gsm, about 350 gsm to about 400 gsm, about 250 gsm to about 260 gsm, about 260 gsm to about 270 gsm, about 270 gsm to about 280 gsm, about 280 gsm to about 290 gsm, about 290 gsm to about 300 gsm, about 300 gsm to about 310 gsm, about 310 gsm to about 320 gsm, about 320 gsm to about 330 gsm, about 330 gsm to about 340 gsm, about 350 gsm, about 350 gsm to about 360 gsm, about 360 gsm to about 370 gsm, about 370 gsm to about 380 gsm, at least about 250 gsm, at least about 260 gsm, at least about 270 gsm, at least about 280 gsm, at least about 290 gsm, at least about 300 gsm, at least about 310 gsm, at least about 320 gsm, at least about 330 gsm, at least about 340 gsm, at least about 350 gsm, at least about 360 gsm, at least about 370 gsm, at least about 380 gsm, less than about 250 gsm, less than about 260 gsm, less than about 270 gsm, less than about 280 gsm, less than about 290 gsm, less than about 300 gsm, less than about 310 gsm, less than about 320 gsm, less than about 330 gsm, less than about 340 gsm, less than about 350 gsm, less than about 360 gsm, less than about 370 gsm, or less than about 380 gsm.

The various embodiments of the first layer 312 and the second layer 314 described herein may be combined without limitation in the base 210 of the fluid collection device 200. For example, embodiments of the second layer 314a described herein may be combined with any one or more of the embodiments of the first layer 312a, 312b, 312c, or 312d.

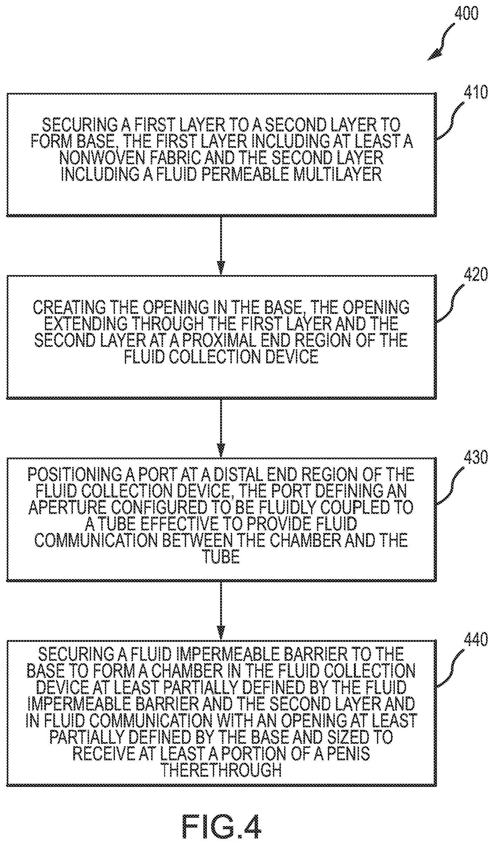

**FIG. 4** is a flow diagram of a method 400 for manufacturing a fluid collection device, according to an embodiment. In an embodiment, the method 400 includes an act 410 of securing a first layer to a second layer to form base. The base formed in the act 410 may include any base described herein, such as a base having the first layer including at least a nonwoven fabric and the second layer including a fluid permeable multilayer. The method 400 also may optionally include an act 420 of creating an opening in the base sized to receive at least a portion of a penis therethrough, the opening extending through the first layer and the second layer at a proximal end region of the fluid collection device. The method 400 also may include an act 430 of positioning a port at a distal end region of the fluid collection device, the port defining an aperture configured to be fluidly coupled to a tube effective to provide fluid communication between the chamber and the tube. The method 400 also may include an act 440 of securing a fluid impermeable barrier to the base to form a chamber in the fluid collection device at least partially defined by the fluid impermeable barrier and the second layer and in fluid communication with the opening at least partially defined by the base.

In some embodiments, the act 410 may include welding the first layer to the second layer with at least one of thermal welding, radio frequency welding, or microwave welding. The first layer and/or the second layer of the base may include any of the first layers and/or the second layers described herein. In some embodiments, the act 440 includes welding the fluid impermeable barrier to the base with at least one of thermal welding, radio frequency welding, or microwave welding. The fluid impermeable barrier may include any of the fluid impermeable barriers described herein.

Acts 410, 420, 430 and 440 of the method 400 are for illustrative purposes. For example, the acts 410, 420, 430 and 440 of the method 400 may be performed in different orders, split into multiple acts, modified, supplemented, or combined. In an embodiment, one or more of the acts 410, 420, 430 and 440 of the method 400 may be omitted from the method 400. Any of the 410, 420, 430 and 440 of the method 400 may include using any of the fluid collection devices or systems disclosed herein.

Also described herein is a method for collecting fluids, according to an embodiment. The method includes an act of positioning a base of a fluid collection device over or around a urethra of a penis of the user. The fluid collection device may include any fluid collection device described herein. In some embodiments, the act may include positioning the base around the urethra of the penis of the user by inserting at least a portion of the penis into an opening in the base. In some embodiments, the act includes positioning the opening of the base over the urethra of the penis that is buried on the user. The method also includes an act of securing the fluid collection device to the user. The act may include securing an adhesive on the base to at least a portion of skin around the penis of the user. The method also may include an act of receiving fluids from the urethra into the chamber of the fluid collection device. The method also includes an act of applying suction effective to suction the fluids from the chamber via a secured thereto.

While various aspects and embodiments have been disclosed herein, other aspects and embodiments are contemplated. The various aspects and embodiments disclosed herein are for purposes of illustration and are not intended to be limiting.

Terms of degree (*e*.*g*., "about," "substantially," "generally," etc.) indicate structurally or functionally insignificant variations. Structurally or functionally insignificant variations are known to a person having ordinary skill in the art. For example, in some embodiments, a person having ordinary skill in the art would understand that structurally or functional insignificant variations may include varying the quantity modified by the term of degree by ± 10%, ±5%, or +2%. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection device (200), comprising:
a fluid impermeable barrier (202) at least partially defining a chamber (316) within the fluid collection device, the fluid impermeable barrier extending at least partially between a proximal end region (216) of the fluid collection device and a distal end region (218a) of the fluid collection device; and
a base (210) secured to the fluid impermeable barrier and at least partially defining the chamber and an opening (218) in fluid communication with the chamber and sized to receive at least a portion of a penis therethrough, the base including at least a first layer (312) including at least a nonwoven fabric extending at least partially between the proximal end region and the distal end region, the first layer including a first inner portion (612b, 712c) and a first outer portion (612a, 712a), the opening being positioned proximate to the proximal end region; and
a port (220) positioned proximate to the distal end region of the fluid collection device, the port defining an aperture (222) configured to be fluidly coupled to a tube effective to provide fluid communication between the chamber and the tube, the fluid collection device (200) being **characterised by**: at least a portion of the fluid impermeable barrier (202) being substantially planar and at least a portion of the base (210) being substantially planar.

2. The fluid collection device of claim 1, wherein the base includes a second layer (314) secured to the first layer and at least partially defining the chamber, the second layer including a fluid permeable multilayer fabric at least partially positioned between the first layer and the chamber and extending at least partially between the proximal end region and the distal end region, the first outer portion of the first layer being positioned on the fluid collection device to be at least proximate to a user, the first inner portion of the first layer being positioned proximate to the second layer, and the opening extending through both the first layer and the second layer.

3. The fluid collection device of claim 2, wherein:
the nonwoven fabric of the first layer includes a spunbonded fabric including polyethylene and polypropylene; the spunbonded fabric has a density of about 20 g/m² to about 100 g/m²; and the first inner portion includes a nonwoven polypropylene.

4. The fluid collection device of claim 2, wherein the first inner portion includes a nonwoven polyester material and the first outer portion includes a polyethylene material, wherein the first layer has a density of about 25 g/m² to about 120 g/m².

5. The fluid collection device of claim 2, wherein the first layer includes a spunbonded fabric, the first inner portion includes a cellulose material and the first outer portion includes polyethylene.

6. The fluid collection device of claim 2, wherein the first inner portion (712c) and the first outer portion (712a) include spunbonded polypropylene, and the first layer (312c) further includes an intermediate portion (712b) including melt blown polypropylene between the first inner portion and the first outer portion, wherein the first layer includes a density of about 12 g/m² to about 60 g/m².

7. The fluid collection device of claim 2, wherein the first inner portion (912e) includes spunbonded polypropylene and the first outer portion (912a) includes nonwoven polyethylene, the first layer (312d) further including a first intermediate portion (912d) including melt blown polypropylene adjacent to the first inner portion, a second intermediate portion (912c) including spunbonded polypropylene adjacent to the first intermediate portion, and a third intermediate portion (912b) including melt blown polypropylene between the second intermediate portion and the first outer portion.

8. The fluid collection device of claim 2, wherein the first inner portion (812d) includes spunbonded polypropylene and the first outer portion (812a) includes nonwoven polyethylene, the first layer (312c) further including a first intermediate portion (812c) including melt blown polypropylene adjacent to the first inner portion and a second intermediate portion (812b) including spunbonded polypropylene between the first intermediate portion and the first outer portion, wherein the first layer includes a density of about 25 g/m² to about 85 g/m².

9. The fluid collection device of any of claims 2-8, wherein:
the first layer is configured to provide venting to the chamber when a vacuum of about 0 Pa (0 mmHg) to about 2666 Pa (20 mmHg) is pulled on the chamber through the aperture; the first layer includes a porous layer having pores of about 0.1 µm to about 3 µm;.
the second layer (314a) includes a spacer fabric including a second inner portion (514c), a second outer portion (514a), and an intermediate portion (514b) connecting the second inner portion and the second outer portion;
the spacer fabric of the second layer includes a polyester spacer fabric; and.
the second inner portion includes an inner mesh portion having holes of a first size and the second outer portion includes an outer mesh portion having holes of a second size smaller than the holes of the first size.

10. The fluid collection device of claim 9, wherein the spacer fabric has a density of about 190 g/m² to about 260 g/m².

11. The fluid collection device of any of claims 2-10, wherein the second layer includes a polyester-spandex knit fabric having a density of about 250 g/m² to about 380 g/m².

12. The fluid collection device of any of claims 2-9, wherein the second layer includes:
a second inner portion including one or more of non-woven polyester, polypropylene, or polyethylene; and
a second outer portion include one or more of polyester or nylon.

13. The fluid collection device of any of claims 2-12, wherein:
the second layer has a thickness of about 0.5 mm to about 10.0 mm;
the second inner portion of the second layer at least partially defines the chamber; and
the first outer portion of the first layer at least partially defines an outer surface of the fluid collection device.

14. A method of manufacturing a fluid collection device according to claim 1, the method comprising:
securing a first layer to a second layer to form a base having at least a portion that is substantially planar, the first layer including at least a nonwoven fabric and the second layer including a fluid permeable multilayer;
securing a fluid impermeable barrier having at least a portion that is substantially planar to the base to form a chamber in the fluid collection device at least partially defined by the fluid impermeable barrier and the second layer and in fluid communication with an opening at least partially defined by the base and sized to receive at least a portion of a penis therethrough, the fluid collection device having a proximal end region and a distal end region, the base and the fluid impermeable barrier extending from the proximal end region to the distal end region of the fluid collection device;
creating the opening in the base, the opening extending through the first layer and the second layer at the proximal end region of the fluid collection device; and
positioning a port at the distal end region of the fluid collection device, the port defining an aperture configured to be fluidly coupled to a tube effective to provide fluid communication between the chamber and the tube.

15. The method of claim 14, wherein:
securing a first layer to a second layer to form a base includes welding the first layer to the second layer with at least one of thermal welding, radio frequency welding, or microwave welding; and
securing a fluid impermeable barrier to the base includes welding the fluid impermeable barrier to the base with at least one of thermal welding, radio frequency welding, or microwave welding.

## Patentansprüche

1. Fluidsammelvorrichtung (200), umfassend:
eine fluidundurchlässige Barriere (202), die zumindest teilweise eine Kammer (316) innerhalb der Fluidsammelvorrichtung definiert, wobei sich die fluidundurchlässige Barriere zumindest teilweise zwischen einem proximalen Endbereich (216) der Fluidsammelvorrichtung und einem distalen Endbereich (218a) der Fluidsammelvorrichtung erstreckt; und
eine Basis (210), die an der fluidundurchlässigen Barriere befestigt ist und mindestens teilweise die Kammer und eine Öffnung (218) in Fluidkommunikation mit der Kammer definiert und so bemessen ist, dass sie mindestens einen Abschnitt eines Penis durch sie hindurch aufnimmt, wobei die Basis mindestens eine erste Schicht (312) enthält, die mindestens einen Vliesstoff enthält, der sich mindestens teilweise zwischen dem proximalen Endbereich und dem distalen Endbereich erstreckt, wobei die erste Schicht einen ersten inneren Abschnitt (612b, 712c) und einen ersten äußeren Abschnitt (612a, 712a) enthält, wobei die Öffnung in der Nähe des proximalen Endbereichs positioniert ist; und
einen Port (220), der in der Nähe des distalen Endbereichs der Fluidsammelvorrichtung positioniert ist, wobei der Port eine Öffnung (222) definiert, die konfiguriert ist, um fluidisch mit einem Rohr gekoppelt zu werden, das wirksam ist, um eine Fluidkommunikation zwischen der Kammer und dem Rohr bereitzustellen, wobei die Fluidsammelvorrichtung (200) **dadurch gekennzeichnet ist, dass**: mindestens ein Abschnitt der fluidundurchlässigen Barriere (202) im Wesentlichen eben ist und mindestens ein Abschnitt der Basis (210) im Wesentlichen eben ist.

2. Fluidsammelvorrichtung nach Anspruch 1, wobei die Basis eine zweite Schicht (314) enthält, die an der ersten Schicht befestigt ist und zumindest teilweise die Kammer definiert, wobei die zweite Schicht ein fluiddurchlässiges mehrschichtiges Gewebe enthält, das zumindest teilweise zwischen der ersten Schicht und der Kammer positioniert ist und sich zumindest teilweise zwischen dem proximalen Endbereich und dem distalen Endbereich erstreckt, wobei der erste äußere Abschnitt der ersten Schicht an der Fluidsammelvorrichtung positioniert ist, um zumindest in der Nähe eines Benutzers zu sein, wobei der erste innere Abschnitt der ersten Schicht in der Nähe der zweiten Schicht positioniert ist und sich die Öffnung sowohl durch die erste Schicht als auch durch die zweite Schicht erstreckt.

3. Fluidsammelvorrichtung nach Anspruch 2, wobei:
der Vliesstoff der ersten Schicht einen Spinnvliesstoff enthält, der Polyethylen und Polypropylen enthält; der Spinnvliesstoff eine Dichte von etwa 20 g/m² bis etwa 100 g/m² aufweist; und der erste innere Abschnitt ein ungewebtes Polypropylen enthält.

4. Fluidsammelvorrichtung nach Anspruch 2, wobei der erste innere Abschnitt ein ungewebtes Polyestermaterial enthält und der erste äußere Abschnitt ein Polyethylenmaterial enthält, wobei die erste Schicht eine Dichte von etwa 25 g/m² bis etwa 120 g/m² aufweist.

5. Fluidsammelvorrichtung nach Anspruch 2, wobei die erste Schicht einen Spinnvliesstoff enthält, der erste innere Abschnitt ein Cellulosematerial enthält und der erste äußere Abschnitt Polyethylen enthält.

6. Fluidsammelvorrichtung nach Anspruch 2, wobei der erste innere Abschnitt (712c) und der erste äußere Abschnitt (712a) spinngebundenes Polypropylen enthalten und die erste Schicht (312c) ferner einen Zwischenabschnitt (712b) enthält, der schmelzgeblasenes Polypropylen zwischen dem ersten inneren Abschnitt und dem ersten äußeren Abschnitt enthält, wobei die erste Schicht eine Dichte von etwa 12 g/m² bis etwa 60 g/m²enthält.

7. Fluidsammelvorrichtung nach Anspruch 2, wobei der erste innere Abschnitt (912e) spinngebundenes Polypropylen enthält und der erste äußere Abschnitt (912a) ungewebtes Polyethylen enthält, wobei die erste Schicht (312d) ferner einen ersten Zwischenabschnitt (912d), der schmelzgeblasenes Polypropylen benachbart zu dem ersten inneren Abschnitt enthält, einen zweiten Zwischenabschnitt (912c), der spinngebundenes Polypropylen benachbart zu dem ersten Zwischenabschnitt enthält, und einen dritten Zwischenabschnitt (912b) enthält, der schmelzgeblasenes Polypropylen zwischen dem zweiten Zwischenabschnitt und dem ersten äußeren Abschnitt enthält.

8. Fluidsammelvorrichtung nach Anspruch 2, wobei der erste innere Abschnitt (812d) spinngebundenes Polypropylen enthält und der erste äußere Abschnitt (812a) ungewebtes Polyethylen enthält, wobei die erste Schicht (312c) ferner einen ersten Zwischenabschnitt (812c), der schmelzgeblasenes Polypropylen benachbart zu dem ersten inneren Abschnitt enthält, und einen zweiten Zwischenabschnitt (812b) enthält, der spinngebundenes Polypropylen zwischen dem ersten Zwischenabschnitt und dem ersten äußeren Abschnitt enthält, wobei die erste Schicht eine Dichte von etwa 25 g/m² bis etwa 85 g/m²enthält.

9. Fluidsammelvorrichtung nach einem der Ansprüche 2-8, wobei:
die erste Schicht so konfiguriert ist, dass sie für eine Belüftung der Kammer sorgt, wenn ein Vakuum von etwa 0 Pa (0 mmHg) bis etwa 2666 Pa (20 mmHg) durch die Öffnung an die Kammer gezogen wird; die erste Schicht enthält eine poröse Schicht mit Poren von etwa 0,1 µm bis etwa 3 µm;
die zweite Schicht (314a) ein Abstandsgewebe enthält, das einen zweiten inneren Abschnitt (514c), einen zweiten äußeren Abschnitt (514a) und einen Zwischenabschnitt (514b) enthält, der den zweiten inneren Abschnitt und den zweiten äußeren Abschnitt verbindet;
das Abstandsgewebe der zweiten Schicht ein Polyester-Abstandsgewebe enthält; und
der zweite innere Abschnitt einen inneren Maschenabschnitt enthält, der Löcher einer ersten Größe aufweist, und der zweite äußere Abschnitt einen äußeren Maschenabschnitt enthält, der Löcher einer zweiten Größe aufweist, die kleiner als die Löcher der ersten Größe ist.

10. Fluidsammelvorrichtung nach Anspruch 9, wobei das Abstandsgewebe eine Dichte von etwa 190 g/m² bis etwa 260 g/m²aufweist.

11. Fluidsammelvorrichtung nach einem der Ansprüche 2-10, wobei die zweite Schicht einen gestrickten Polyester-Spandex-Stoff mit einer Dichte von etwa 250 g/m² bis etwa 380 g/m²enthält.

12. Fluidsammelvorrichtung nach einem der Ansprüche 2-9, wobei die zweite Schicht Folgendes enthält:
einen zweiten inneren Abschnitt, der eines oder mehrere von ungewebtem Polyester, Polypropylen oder Polyethylen enthält; und
ein zweiter äußerer Abschnitt eines oder mehrere von Polyester oder Nylon enthält.

13. Fluidsammelvorrichtung nach einem der Ansprüche 2-12, wobei:
die zweite Schicht eine Dicke von etwa 0,5 mm bis etwa 10,0 mm aufweist;
der zweite innere Abschnitt der zweiten Schicht zumindest teilweise die Kammer definiert; und
der erste äußere Abschnitt der ersten Schicht zumindest teilweise eine Außenfläche der Fluidsammelvorrichtung definiert.

14. Verfahren zum Herstellen einer Fluidsammelvorrichtung nach Anspruch 1, wobei das Verfahren Folgendes umfasst:
Befestigen einer ersten Schicht an einer zweiten Schicht, um eine Basis zu bilden, die mindestens einen Abschnitt aufweist, der im Wesentlichen eben ist, wobei die erste Schicht mindestens einen Vliesstoff enthält und die zweite Schicht eine fluiddurchlässige Mehrschicht enthält;
Befestigen einer fluidundurchlässigen Barriere mit mindestens einem Abschnitt, der im Wesentlichen eben zu der Basis ist, um eine Kammer in der Fluidsammelvorrichtung zu bilden, die mindestens teilweise durch die fluidundurchlässige Barriere und die zweite Schicht definiert ist und in Fluidkommunikation mit einer Öffnung steht, die mindestens teilweise durch die Basis definiert ist und so bemessen ist, dass sie mindestens einen Abschnitt eines Penis durch sie hindurch aufnimmt, wobei die Fluidsammelvorrichtung einen proximalen Endbereich und einen distalen Endbereich aufweist, wobei sich die Basis und die fluidundurchlässige Barriere von dem proximalen Endbereich zu dem distalen Endbereich der Fluidsammelvorrichtung erstrecken;
Erzeugen der Öffnung in der Basis, wobei sich die Öffnung durch die erste Schicht und die zweite Schicht an dem proximalen Endbereich der Fluidsammelvorrichtung erstreckt; und
Positionieren eines Ports an dem distalen Endbereich der Fluidsammelvorrichtung, wobei der Port eine Öffnung definiert, die konfiguriert ist, um fluidisch mit einem Rohr gekoppelt zu werden, das wirksam ist, um eine Fluidkommunikation zwischen der Kammer und dem Rohr bereitzustellen.

15. Verfahren nach Anspruch 14, wobei:
das Befestigen einer ersten Schicht an einer zweiten Schicht, um eine Basis zu bilden, das Verschweißen der ersten Schicht mit der zweiten Schicht mit mindestens einem von thermischem Schweißen, Hochfrequenzschweißen oder Mikrowellenschweißen einschließt; und
das Befestigen einer fluidundurchlässigen Barriere an der Basis das Verschweißen der fluidundurchlässigen Barriere mit der Basis mit mindestens einem von thermischem Schweißen, Hochfrequenzschweißen oder Mikrowellenschweißen einschließt.

## Revendications

1. Dispositif de collecte des fluides (200), comprenant :
une barrière imperméable aux fluides (202) définissant au moins partiellement une chambre (316) à l'intérieur du dispositif de collecte des fluides, la barrière imperméable aux fluides s'étendant au moins partiellement entre une région d'extrémité proximale (216) du dispositif de collecte des fluides et une région d'extrémité distale (218a) du dispositif de collecte des fluides ; et
une base (210) fixée à la barrière imperméable aux fluides et définissant au moins partiellement la chambre et une ouverture (218) en communication fluidique avec la chambre et dimensionnée pour recevoir au moins une partie d'un pénis à travers celle-ci, la base comprend au moins une première couche (312) comprenant au moins un tissu non tissé s'étendant au moins partiellement entre la région d'extrémité proximale et la région d'extrémité distale, la première couche comprenant une première partie intérieure (612b, 712c) et une première partie extérieure (612a, 712a), l'ouverture étant positionnée à proximité de la région d'extrémité proximale ; et
un orifice (220) positionné à proximité de la région de l'extrémité distale du dispositif de collecte des fluides, l'orifice définissant une ouverture (222) configurée pour être couplée fluidiquement à un tube efficace pour fournir une communication fluidique entre la chambre et le tube, le dispositif de collecte des fluides (200) étant **caractérisé par** : au moins une partie de la barrière imperméable aux fluides (202) étant sensiblement plane et au moins une partie de la base (210) étant sensiblement plane.

2. Dispositif de collecte des fluides selon la revendication 1, dans lequel la base comprend une seconde couche (314) fixée à la première couche et définissant au moins partiellement la chambre, la seconde couche comprenant un tissu multicouche perméable aux fluides au moins partiellement positionné entre la première couche et la chambre et s'étendant au moins partiellement entre la région d'extrémité proximale et la région d'extrémité distale, la première partie extérieure de la première couche étant positionnée sur le dispositif de collecte des fluides au moins à proximité d'un utilisateur, la première partie intérieure de la première couche étant positionnée à proximité de la seconde couche, et l'ouverture s'étendant à la fois à travers la première couche et la seconde couche.

3. Dispositif de collecte des fluides selon la revendication 2, dans lequel :
le tissu non tissé de la première couche comprend un tissu filé-lié comprenant du polyéthylène et du polypropylène ; le tissu filé-lié a une densité d'environ 20 g/m² à environ 100 g/m²; et la première partie intérieure comprend un polypropylène non tissé.

4. Dispositif de collecte des fluides selon la revendication 2, dans lequel la première partie intérieure comprend un matériau polyester non tissé et la première partie extérieure comprend un matériau polyéthylène, dans lequel la première couche a une densité d'environ 25 g/m² à environ 120 g/m².

5. Dispositif de collecte des fluides selon la revendication 2, dans lequel la première couche comprend un tissu filé-lié, la première partie intérieure comprend un matériau cellulosique et la première partie extérieure comprend du polyéthylène.

6. Dispositif de collecte des fluides selon la revendication 2, dans lequel la première partie intérieure (712c) et la première partie extérieure (712a) comprennent du polypropylène filé-lié, et la première couche (312c) comprend en outre une partie intermédiaire (712b) comprenant du polypropylène soufflé par fusion entre la première partie intérieure et la première partie extérieure, dans laquelle la première couche comprend une densité d'environ 12 g/m² à environ 60 g/m².

7. Dispositif de collecte des fluides selon la revendication 2, dans lequel la première partie intérieure (912e) comprend du polypropylène filé-lié et la première partie extérieure (912a) comprend du polyéthylène non tissé, la première couche (312d) comprenant en outre une première partie intermédiaire (912d) comprenant du polypropylène soufflé à chaud adjacent à la première partie intérieure, une deuxième partie intermédiaire (912c) comprenant du polypropylène filé-lié adjacent à la première partie intermédiaire, et une troisième partie intermédiaire (912b) comprenant du polypropylène soufflé à chaud entre la deuxième partie intermédiaire et la première partie extérieure.

8. Dispositif de collecte des fluides selon la revendication 2, dans lequel la première partie intérieure (812d) comprend du polypropylène filé-lié et la première partie extérieure (812a) comprend du polyéthylène non tissé, la première couche (312c) comprenant en outre une première partie intermédiaire (812c) comprenant du polypropylène soufflé à chaud adjacent à la première partie intérieure et une deuxième partie intermédiaire (812b) comprenant du polypropylène filé-lié entre la première partie intermédiaire et la première partie extérieure, dans laquelle la première couche comprend une densité d'environ 25 g/m² à environ 85 g/m².

9. Dispositif de collecte des fluides selon l'une quelconque des revendications 2 à 8, dans lequel :
la première couche est configurée pour fournir une ventilation à la chambre lorsqu'un vide d'environ 0 Pa (0 mmHg) à environ 2666 Pa (20 mmHg) est tiré sur la chambre à travers l'ouverture ; la première couche comprend une couche poreuse ayant des pores d'environ 0,1 µm à environ 3 µm ;
la seconde couche (314a) comprend un tissu d'espacement avec une seconde partie intérieure (514c), une seconde partie extérieure (514a) et une partie intermédiaire (514b) reliant la seconde partie intérieure et la seconde partie extérieure ;
le tissu d'espacement de la seconde couche comprend un tissu d'espacement en polyester ; et
la seconde partie intérieure comprend une partie de maille intérieure avec des trous d'une première taille et la seconde partie extérieure comprend une partie de maille extérieure avec des trous d'une seconde taille plus petite que les trous de la première taille.

10. Dispositif de collecte des fluides selon la revendication 9, dans lequel le tissu d'espacement a une densité d'environ 190 g/m² à environ 260 g/m².

11. Dispositif de collecte des fluides selon l'une quelconque des revendications 2 à 10, dans lequel la seconde couche comprend un tricot en polyester-spandex ayant une densité d'environ 250 g/m² à environ 380 g/m².

12. Dispositif de collecte des fluides selon l'une quelconque des revendications 2 à 9, dans lequel la seconde couche comprend :
une seconde partie intérieure comprenant un ou plusieurs polyester non tissés, du polypropylène ou du polyéthylène ; et
une seconde partie extérieure comprenant un ou plusieurs polyester ou nylon.

13. Dispositif de collecte des fluides selon l'une quelconque des revendications 2 à 12, dans lequel :
la seconde couche a une épaisseur d'environ 0,5 mm à environ 10,0 mm ;
la seconde partie intérieure de la seconde couche définit au moins partiellement la chambre ; et
la première partie extérieure de la première couche définit au moins partiellement une surface extérieure du dispositif de collecte des fluides.

14. Procédé de fabrication d'un dispositif de collecte des fluides selon la revendication 1, le procédé comprenant :
la fixation d'une première couche à une seconde couche pour former une base dont au moins une partie est sensiblement plane, la première couche comprenant au moins un tissu non tissé et la seconde couche comprenant une multicouche perméable aux fluides ;
la fixation d'une barrière imperméable aux fluides dont au moins une partie est sensiblement plane par rapport à la base pour former une chambre dans le dispositif de collecte des fluides au moins partiellement définie par la barrière imperméable aux fluides et la seconde couche et en communication fluidique avec une ouverture au moins partiellement définie par la base et dimensionnée pour recevoir au moins une partie d'un pénis à travers celle-ci, le dispositif de collecte des fluides ayant une région d'extrémité proximale et une région d'extrémité distale, la base et la barrière imperméable aux fluides s'étendant de la région d'extrémité proximale à la région d'extrémité distale du dispositif de collecte des fluides ;
la création de l'ouverture dans la base, l'ouverture s'étendant à travers la première couche et la seconde couche au niveau de la région d'extrémité proximale du dispositif de collecte des fluides ; et
le positionnement d'un orifice au niveau de la région d'extrémité distale du dispositif de collecte des fluides, l'orifice définissant une ouverture configurée pour être couplée fluidiquement à un tube efficace pour fournir une communication fluidique entre la chambre et le tube.

15. Procédé selon la revendication 14, dans lequel :
la fixation d'une première couche à une seconde couche pour former une base comprend le soudage de la première couche à la seconde couche par au moins l'un des procédé suivants : soudage thermique, soudage par radiofréquence ou soudage par micro-ondes ; et
la fixation d'une barrière imperméable aux fluides à la base comprend le soudage de la barrière imperméable aux fluides à la base à l'aide d'au moins un des procédés suivants : soudage thermique, soudage par radiofréquence ou soudage par micro-ondes.
